(19) Europäisches Patentamt / European Patent Office / Office européen des brevets

(11) **EP 3 311 747 B1**

(12) **EUROPEAN PATENT SPECIFICATION**

| | |
|---|---|
| (45) Date of publication and mention of the grant of the patent:<br>**04.09.2019 Bulletin 2019/36** | (51) Int Cl.:<br>***A61B 6/00*** *(2006.01)* |

(21) Application number: **17197282.1**

(22) Date of filing: **19.10.2017**

(54) **TORQUE ASSISTING DEVICE, METHOD AND MAMMARY MACHINE**

DREHMOMENTUNTERSTÜTZUNGSVORRICHTUNG, VERFAHREN UND MAMILLÄRE MASCHINE

DISPOSITIF D'ASSISTANCE DE COUPLE, PROCÉDÉ ET MACHINE MAMMAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.10.2016 CN 201610912836**

(43) Date of publication of application:
**25.04.2018 Bulletin 2018/17**

(73) Proprietor: **Shenyang Neusoft Medical Systems Co., Ltd.**
**Shenyang 110167 (CN)**

(72) Inventor: **SUN, Junjie**
**Shenyang, Liaoning 110167 (CN)**

(74) Representative: **Grünecker Patent- und Rechtsanwälte**
**PartG mbB**
**Leopoldstraße 4**
**80802 München (DE)**

(56) References cited:
**US-A- 5 109 571**      **US-A- 5 388 141**
**US-A1- 2011 062 837**      **US-A1- 2015 145 397**

# Description

## BACKGROUND

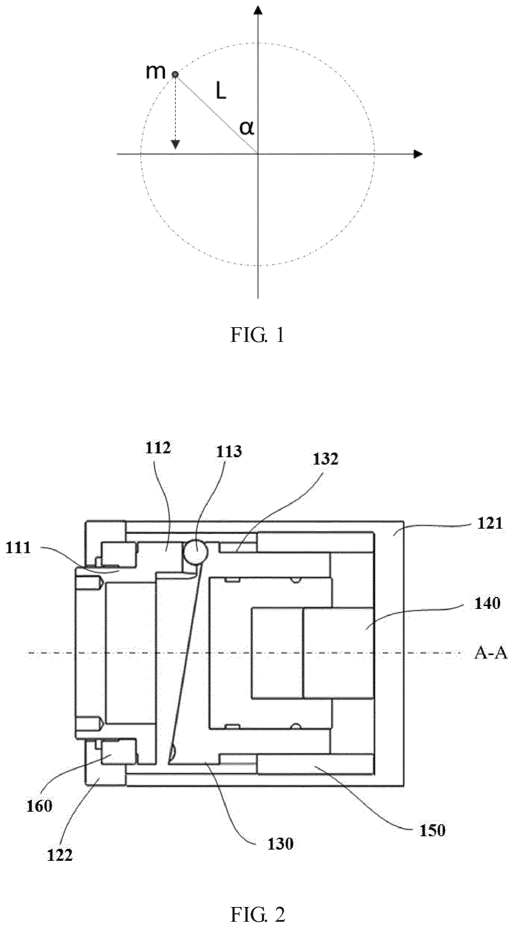

When a rigid load rotates about an axis, the gravity of the load will generate a torque if the center of gravity of the load does not coincide with the axis of rotation. Both a magnitude and direction of the torque vary with rotation angle and rotation phase of the load. Referring to FIG. 1, a mass of the load is m, a radius from the centre of gravity of the load to the axis of rotation is L, an included angle between a real-time radius from the centre of gravity of the load to the axis of rotation and a vertical direction is $\alpha$ when the load is in rotation, and the torque generated by the gravity of the load is $M_G$:

$$M_G = m \times g \times L \times \sin\alpha \quad (1)$$

where g is a gravitational acceleration.

It may be seen from the formula (1) that the magnitude of the torque generated by the gravity of the load varies in the range of 0 to $m \times g \times L$.

The direction of the torque generated by the gravity of the load is clockwise when the load is in a first and a fourth quadrant and is counter-clockwise when the load is in a second and a third quadrant. In some cases, the torque generated by the gravity of the load when the load rotates about the axis needs counteracting.

US 2011/062837 A1 discloses a hinge unit including an elastic member, and a cam unit, where the cam unit includes a shaft, a rotational cam and a reciprocating cam connected to the shaft so that rotation thereof is restricted, and deforming the elastic member as moved by the rotational cam along the shaft. The cam unit converts a restoring force exerted from the elastic member to a rotational force so that a washing machine provided with the hinge unit may be easily opened with less force and closed with less impact on a top cover by reducing rotation speed.

US 2015/145397 A1 discloses a home appliance including a cam assembly. The cam assembly includes a first cam configured to rotate with a door of the home appliance, and a second cam configured to linearly move via rotation of the first cam. The home appliance further includes a spring configured to be compressed by the horizontal linear movement of the cam assembly. Different moments are generated by the cam assembly in the respective rotation angle of the door to restrict the rotation of the door based on the comfort of the user at a corresponding opening degree of the door.

US 5,109,571 A discloses a door comprising a pair of movable members arranged within a casing for being rotated with a rotary shaft to be rigidly fitted to a swing door. The hinge further comprising a movable cam so arranged as to be rotatable with and axially slidable relative to the rotary shaft and a fixed cam so arranged as to be capable of being engaged with the movable cam that a torque is generated within the hinge to rotate the rotary shaft in one direction within a given range of rotation of the rotary shaft.

US 5,388,141 A discloses an X-ray apparatus comprising an apparatus section which is pivotable about a horizontal pivotal axis that does not extend through the centre of gravity of the apparatus section, and also comprising a spring assembly for compensation of the torque which is exerted by the weight of the apparatus section on a coupling member which moves in an orbit about the pivotal axis during pivoting of the apparatus section. The centre of the orbit is situated outside the pivotal axis in a vertical plane containing the pivotal axis, and the coupling member is guided in a guide device which is connected to the apparatus section and which extends towards the pivotal axis. In this way, suitable counterbalancing is achieved for a comparatively wide range of pivotal angles.

NEUSOFT MEDICAL SYSTEMS CO., LTD. (NMS), founded in 1998 with its world headquarters in China, is a leading supplier of medical equipment, medical IT solutions, and healthcare services. NMS supplies medical equipment with a wide portfolio, including CT, Magnetic Resonance Imaging (MRI), digital X-ray machine, ultrasound, Positron Emission Tomography (PET), Linear Accelerator (LINAC), and biochemistry analyser. Currently, NMS' products are exported to over 60 countries and regions about the globe, serving more than 5,000 renowned customers. NMS's latest successful developments, such as 128 Multi-Slice CT Scanner System, Superconducting MRI, LINAC, and PET products, have led China to become a global high-end medical equipment manufacturer. As an integrated supplier with extensive experience in large medical equipment, NMS has been committed to the study of avoiding secondary potential harm caused by excessive X-ray irradiation to the subject during the CT scanning process.

## BRIEF DESCRIPTION OF DRAWINGS

FIG. 1 is a schematic diagram of a position of a centre of gravity of a rigid member when the rigid member rotates about an axis.

FIG. 2 is a cross-sectional diagram of a torque assisting device according to an example of the present disclosure.

FIG. 3 is a perspective diagram of a rotating mechanism according to an example of the present disclosure.

FIG. 4 is a perspective diagram of a fixing cylinder according to an example of the present disclosure.

FIG. 5 is a perspective diagram of another fixing cylinder according to an example of the present disclosure.

FIG. 6 is a perspective diagram of the fixing cylinder in FIG. 5 in another direction.

FIG. 7 is a perspective diagram of a nitrogen spring according to an example of the present disclosure.

FIG. 8 is a schematic diagram of a curve relationship between a rotation angle of a rotating mechanism and a compression stroke of a gas spring according to an example of the present disclosure, in which an inclining rate of the curve also reflects an inclination angle of each point on a motion trajectory corresponding to the rotation angle.

FIG. 9 is a schematic diagram of a curve relationship between a rotation angle of a rotating mechanism and a compression stroke of a gas spring according to an example of the present disclosure, which shows forces exerted on a rigid member.

FIG. 10 is a flow chart of a torque assisting method of a torque assisting device according to an example of the present disclosure.

FIG. 11 is a motion state diagram of a torque assisting device according to an example of the present disclosure, which shows positional relationship of different components when a rigid member is at a low point of a motion trajectory.

FIG. 12 is a motion state diagram of a torque assisting device according to an example of the present disclosure, which shows positional relationships of different components when a rigid member is between a low point and a high point of a motion trajectory.

FIG. 13 is a motion state diagram of a torque assisting device according to an example of the present disclosure, which shows positional relationships of different components when a rigid member is at a high point of a motion trajectory.

FIG. 14 is a motion state diagram of a torque assisting device according to an example of the present disclosure, which shows positional relationships of different components when a rigid member is between a high point and a low point of a motion trajectory.

FIG. 15 is a cross-sectional diagram of a torque assisting device according to another example of the present disclosure.

FIG. 16 is a perspective diagram of a rotating mechanism according to another example of the present disclosure.

FIG. 17 is a perspective diagram of a fixing cylinder according to another example of the present disclosure.

FIG. 18 is a motion state diagram of a torque assisting device according to another example of the present disclosure, which shows positional relationships of different components when a rigid member is at a low point of a motion trajectory.

FIG. 19 is a motion state diagram of a torque assisting device according to another example of the present disclosure, which shows positional relationships of different components when a rigid member is between a low point and a high point of a motion trajectory.

FIG. 20 is a motion state diagram of a torque assisting device according to another example of the present disclosure, which shows positional relationships of different components when a rigid member is at a high point of a motion trajectory.

FIG. 21 is a motion state diagram of a torque assisting device according to an example of the present disclosure, which shows positional relationships of different components when a rigid member is between a high point and a low point of a motion trajectory.

References: A-A: axis of rotation; 110: rotating mechanism; 111: rotating part; 112: support frame; 113: rigid member; 114: clamping groove; 121: cylinder body; 122: cylinder head; 130: fixing cylinder; 131: motion trajectory 132: guide groove, 140: force-applying mechanism, 150: guide post, 160: bearing. B-B: axis of rotation; 210: rotating mechanism; 211: rotating part; 212: motion trajectory; 221: cylinder body; 222: cylinder head; 230: fixing cylinder; 231: rigid member; 232: guide groove; 233: support frame; 234: clamping groove, 240: force-applying mechanism, 250: guide post, 260: bearing.

## DETAILED DESCRIPTION

Hereinafter, an exemplary example will be described in detail, examples of which are shown in the drawings. In the following description, when the drawings are referred to, the same numerals in the different drawings denote the same or similar elements unless otherwise indicated. The implementation described in the following exemplary examples is not representative of all implementations coincident with the present disclosure. Rather, they are merely examples of devices and methods coincident with some aspects of the present disclosure as detailed in the appended claims.

For example, when a load, such as a rotating part of a mammary machine, rotates about an axis, a centre of gravity of the rotating part may not overlap with the axis of rotation, and the gravity of the rotating part will generate a torque $M_G$ so that the rotating part rotates in an unbalanced manner. Therefore, the torque $M_G$ generated by the gravity of the rotating part when the rotating part rotates about the axis is to be eliminated.

A manner of controlling an output of a motor, a counterweight manner or a gas spring assisting manner is generally used to counteract the torque generated by the gravity of the load when the load rotates about the axis.

When the manner of controlling an output of a motor is used, the load is driven by the motor to rotate, and the output torque and speed of the motor are controlled to counteract the torque generated by the gravity of the load. However, a high demand is raised for a driving power and a control precision of the motor, and it is difficult to satisfy the demand.

When a counterweight manner is used, the counterweight is added so that a centre of gravity of the counterweight and the centre of gravity of the load are symmetrical about the axis of rotation. However, drawbacks of this manner are that the mass of the added counterweight is generally large and a large amount of space is occupied. Besides, other functions of the device will be adversely influenced, for example, the gravity of the counterweight makes it harder for the device to rotate upward.

When a gas spring or the like is used as an assisting mechanism, a torque generated by the assisting mechanism and the torque generated by the gravity of the load may be asynchronous, and only a part of the torque generated by the gravity of the load may be counteracted at all angles.

FIG. 2 is a cross-sectional diagram of a torque assisting device according to an example of the present disclosure. The torque assisting device may be used in a mammary machine to counteract the torque $M_G$ generated by the gravity of the rotating part of the mammary machine when the rotating part rotates about the axis to assist the rotating part in always rotating about a fixed axis.

As shown in FIG. 2 and FIGs. 4-6, the torque assisting device may include a rotating mechanism 110, a fixing cylinder 130, and a force-applying mechanism 140. Where the rotating mechanism 110 is provided with a load (not shown in the drawings). The fixing cylinder 130 has one end surface provided with a motion trajectory 131 having an inclination angle which is set according to the torque generated by the gravity of the load. The fixing cylinder 130 is connected with the force-applying mechanism 140. In some cases, one end of the fixing cylinder 130 is provided with the motion trajectory 131 and the other end is connected with the force-applying mechanism 140. The force-applying mechanism 140 may be elastic under an external force, for example, the force-applying mechanism 140 may be moved axially. The rotating mechanism 110 is abutted on the motion trajectory 131 at one end thereof and rotates about the axis of rotation A-A along the motion trajectory 131, i.e., the rotating mechanism 110 is always in contact with the fixing cylinder 130 during rotation.

It is to be noted that when the torque assisting device is used for a mammary machine, the load is a rotating part of the mammary machine.

Since the rotating mechanism 110 itself is symmetrical, the centre of gravity of the rotating mechanism 110 is located approximately on the axis of rotation A-A, so that the influence of the gravity of the rotating mechanism 110 may be ignored, i.e., the torque generated by the gravity of the rotating mechanism 110 is negligible. However, since the centre of gravity of the load on the rotating mechanism 110 is not located on the axis of rotation A-A, the gravity of the load (i.e., mg, where m is a mass of the load and g is the gravitational acceleration) will generate a torque $M_G$ so that the load rotates in an unbalanced manner.

Referring to FIGs. 2, 3 and 4, the rotating mechanism 110 may include a rotating part 111, a support frame 112 provided on the rotating part 111, and a rigid member 113 fixed to the support frame 112. The rigid member 113 is abutted on the motion trajectory 131 of the fixing cylinder 130 to achieve the transfer of force, thereby urging the fixing cylinder 130 to move axially and compressing the force-applying mechanism 140.

Alternatively, the rigid member 113 may be in a point contact or a line contact with the motion trajectory 131.

In the present example, the support frame 112 may protrude from the rotating part 111 to prevent the rotating part 111 from colliding with the fixing cylinder 130 during rotation.

Alternatively, the rotating part 111 and the support frame 112 may be integrally molded, so that the rotating mechanism 110 is simpler in structure.

The rigid member 113 may be a steel ball, a roller or a non-roller body. The present example will be further described with a steel ball as the rigid member 113.

To better fix the steel ball and prevent the steel ball from falling during rotation about the axis of rotation A-A, the support frame 112 is provided with a clamping groove 114 for clamping the steel ball.

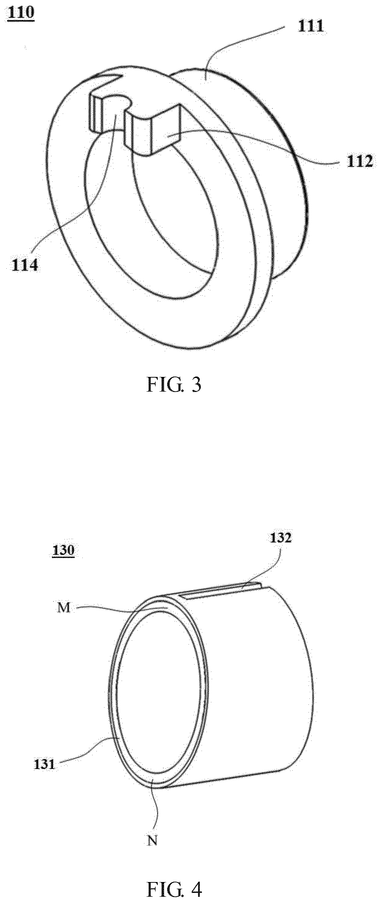

In conjunction with FIGs. 2 and 3, the rotating part 111 is connected with a bearing 160, and the rotating mechanism 110 is fixed and rotatable by the engagement of the bearing 160 and the rotating part 111.

Referring to FIGs. 4 to 6, the fixing cylinder 130 is substantially a cylinder and the axis of rotation A-A is the central axis of the cylinder. The motion trajectory 131 is provided on one end surface of the cylinder, and vertical distances from each point of the motion trajectory 131 to the axis of rotation A-A are r, that is, the centre position of the motion trajectory 131 is located on the axis of rotation A-A. The rotating mechanism 110 rotates about the axis of rotation A-A along the motion trajectory 131.

The motion trajectory 131 is closed, and a projection of the motion trajectory 131 in a circumferential direction of the fixing cylinder 130 is a circular trajectory. The centre of the circular trajectory is the centre position of the motion trajectory 131, and a radius of the circular trajectory is r.

In the present example, the load is driven by the rotating mechanism 110 to rotate about the axis of rotation A-A, where a vertical distance from the centre of gravity of the load to the axis of rotation A-A is L.

The steel ball is abutted on the motion trajectory 131 and rotates about the axis of rotation A-A along the motion trajectory 131, i.e., the steel ball may be always in contact with the fixing cylinder 130 during the rotational movement so that the fixing cylinder 130 is able to transfer a thrust force applied by the force-applying mechanism 140 to the steel ball.

Alternatively, the motion trajectory 131 may be provided with a trajectory groove (not shown in the drawings) engaged with the steel ball so that the steel ball may rotate along the set motion trajectory 131 more easily and is less likely to fall out, and the trajectory groove is provided in such a way that the stress may be adequately transferred to the steel ball.

FIG. 4 is a perspective diagram of a fixing cylinder 130 according to an example. The highest point M (relative to the direction shown in FIG. 4) on the motion trajectory 131 is defined as a low point of the motion trajectory 131, and the lowest point N (relative to the direction shown in FIG. 4) is defined as a high point of the motion trajectory 131. The rotation angle of the centre of gravity of the load is $\alpha$, and the inclination angle of the motion trajectory 131 (i.e., the angle at which each rotation angle $\alpha$ rises, or a complement angle of the included angle between the tangent of the motion trajectory 131 and the axial direction of the fixing cylinder 130) is $\beta$ when the steel ball rotates along the motion trajectory 131.

In the present example, the rotation angle $\alpha$ of the centre of gravity of the load is equal to the rotation angle of the steel ball to better counteract the torque generated by the gravity of the load. Specifically, it is defined that at the low point M the rotation angle $\alpha$ of the centre of gravity of the load is 0° (or 360°); at the high point N the rotation angle $\alpha$ of the centre of gravity of the load is 180°; and when the steel ball rotates from the low point M about the motion trajectory 131 by one turn and then returns to the low point M, $\alpha$=360°.

From the low point M to the high point N, i.e., when the rotation angle $\alpha$ of the centre of gravity of the load is from 0° (or 360°) to 180°, $\beta$ is calculated according to the formula:

$$\beta = \arctan\left(\frac{m \times g \times L \times \sin\alpha}{F \times r}\right) \quad (2)$$

In the formula (2), m is the mass of the load;

g is gravitational acceleration;
L is a vertical distance from the centre of gravity of the load to the axis of rotation A-A;
$\alpha$ is the rotation angle of the centre of gravity of the load;
r is a vertical distance from the motion trajectory 131 to the axis of rotation A-A; and
F is an axial thrust force applied to the fixing cylinder 130 by the force-applying mechanism 140.

Where the direction of the thrust force F is toward the rotating mechanism 110, and the thrust force F changes regularly. For example, the thrust force F is a linearly varying force, and the vertical distance from the action point of the thrust force F to the axis of rotation A-A is equal to the vertical distance from the motion trajectory 131 to the axis of rotation A-A, that is, the vertical distance from the action point of the thrust force F to the axis of rotation A-A is also r.

In addition, if the rotation angle $\alpha$ is a part of a complete cycle, for example between +90° and -90°, the motion trajectory 131 of the steel ball may be divided into two symmetrical parts in a turn (i.e., 360°) correspondingly.

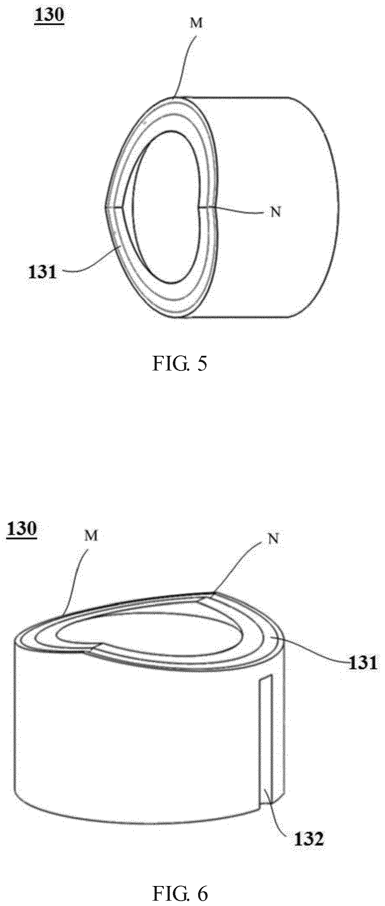

FIGs. 5 and 6 show a perspective diagram of a fixing cylinder 130 according to another example.

Referring to FIG. 5, correspondingly, the rotation angle $\alpha$ is between $\pm$ 90°, and the motion trajectory 131 is provided with two highest points N and two lowest points M. A complete motion trajectory 131 may be used to maintain the balance of the overall structure of the torque assisting device. Of course, only one part (from the highest point N to the lowest point M or from the lowest point M to the highest point N) of the motion trajectory 131 may be used, for example, the part between the highest point N and the lowest point M at the left side in FIG. 5 (relative to the direction shown in FIG. 5).

If it is desired that the rotation angle $\alpha$ is less than 60°, the motion trajectory 131 is provided with three highest points and three lowest points, and only one or two parts in the motion trajectory 131 may be used. Of course, a complete motion trajectory 131 may be used to maintain the balance of the overall structure of the torque assisting device.

Similarly, a corresponding number of highest points and lowest points of the motion trajectory 131 may be set so long as the rotation angle $\alpha$ is an arbitrary angle within $\pm$180°.

When a part of angles (such as, 0-30°, 0-60° and 0-90°) are used as the rotation angle $\alpha$, the inclination angle $\beta$ of the motion trajectory 131 may be calculated in a manner like that when $\alpha$ lies between 0° and 180°, which may be specifically referred to Formula (2).

A torque assisting device of the present example further includes a cylinder body 121. The cylinder body 121 is substantially a cylinder, which is open at one end thereof and is closed at the other end thereof. A cylinder head 122 is provided at the opening of the cylinder body 121. The force-applying mechanism 140 is fixedly connected with an inner wall of the cylinder body 121. The bearing 160 is sandwiched between the rotating part 111 and the cylinder head 122 and enables the rotating mechanism 110 to withstand an axial force and a radial force.

The rotating mechanism 110, the fixing cylinder 130 and the force-applying mechanism 140 are placed in the cylinder body 121 in sequence from the opening position of the cylinder body 121 in this order to reduce external interference.

The inner wall of the cylinder body 121 is provided with a guide post 150. The fixing cylinder 130 is provided with a guide groove 132 slidably engaged with the guide post 150 where the guide post 150 is slidably engaged with the guide groove 132 axially. In an example, the guide post 150 is disposed axially and the guide groove 132 is disposed axially on the fixing cylinder 130 so that the fixing cylinder 130 is moved axially along the guide post 150 and prevented from rotating when the rotating mechanism 110 is in rotation.

Alternatively, the guide groove 132 may be provided on an outer wall of the fixing cylinder 130 so that the fixing cylinder 130 is stably moved axially.

The guide groove 132 may be also provided on the inner wall or the end surface of the fixing cylinder 130 so long as the guide groove 132 is disposed axially.

The number of the guide posts 150 may be set according to the actual demand, and one or more guide posts 150 may be selected.

In an example, there are at least two guide posts 150 which are evenly distributed along the circumference of the fixing cylinder 130 so that the force on the fixing cylinder 130 is more balanced, therefore the fixing cylinder 130 is further stabilized to move axially, and prevented from rotating. Correspondingly, at least two guide grooves 132 are provided on the outer side wall of the fixing cylinder 130, and the at least two guide grooves 132 correspond to the guide posts 150. The fixing cylinder 130 is sandwiched between the at least two guide posts 150 and may be axially translated stably along the guide post 150 under the action of an external force. Moreover, the fixing cylinder 130 is prevented from rotating so that the fixing cylinder 130 may transfer the thrust force F to the steel ball better.

In another example, the inner wall of the cylinder body 121 is provided with the guide groove 132. The guide post 150 is provided on the fixing cylinder 130. The guide post and the guide groove are slidably engaged axially so that when the rotating mechanism 110 rotates, the fixing cylinder 130 is moved axially along the guide post 150. Alternatively, the guide post 150 may be provided on the outer wall or end face or inner wall of the fixing cylinder 130.

In the present example, the force-applying mechanism 140 may be selected according to an actual demand. To reduce an external input, the present example selects an energy storage mechanism as the force-applying mechanism, and the energy storage mechanism may apply a regular and relatively stable axial thrust force F to the fixing cylinder 130.

Alternatively, the force-applying mechanism may be selected as a compressible gas spring. Where the force output by the gas spring does not change by more than 20%, which is relatively stable.

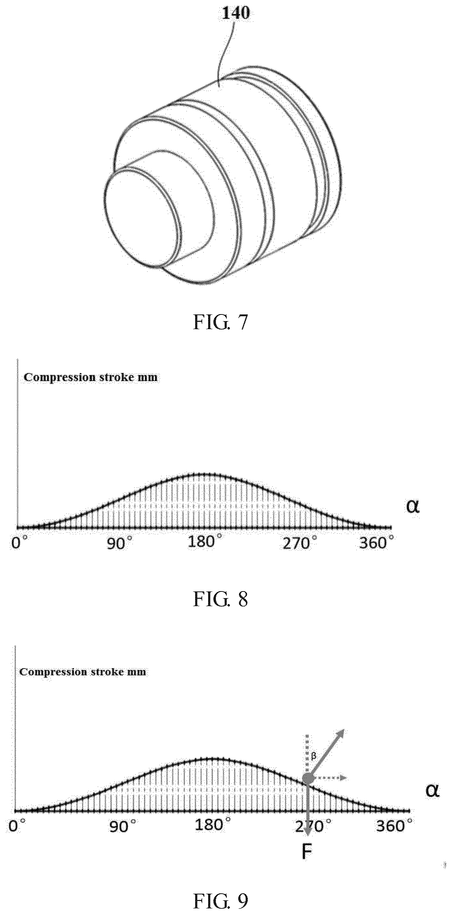

When the rotating mechanism 110 rotates, the thrust force F is brought into contact with the motion trajectory 131 by the transfer action of the fixing cylinder 130. Referring to FIG. 9, the thrust force F and the support force (i.e., the force inclining upward in FIG. 9 which is perpendicular to the support surface of the steel ball) of the surface of the motion trajectory 131 whose inclination angle is β generate a resultant force F × tanβ (i.e., the force points toward the right in FIG. 9). The resultant force and r (the vertical distance from the action point of thrust force F to the axis of rotation A-A) will generate a moment $M_F$,

$$M_F = F \times \tan\beta \times r.$$

In an example, after the magnitude of the torque generated by the gravity of the load is determined, the magnitude of the thrust force F may be selected, i.e., the magnitude of the thrust force F is determined by the gravity of the load.

Referring to FIG. 7, to counteract the torque $M_G$ generated by the gravity of the load, the force-applying mechanism 140 may be selected as a nitrogen spring. Also, the nitrogen spring outputs a relatively stable thrust force F to accurately calculate the value of β.

The thrust force F of the nitrogen spring is calculated according to the formula:

$$F = F_{initial} + s \times K \ (3)$$

In the Formula (3), $F_{initial}$ is a value of an initial output force of the nitrogen spring, s is a movement stroke of the nitrogen spring, and K is an elastic coefficient of the nitrogen spring.

In an example, a process of transferring the thrust force F is as follows: assuming that the thrust force applied to the fixing cylinder 130 by the nitrogen spring is $F_1$, at the same time the axial force at the position where the steel ball is in contact with the fixing cylinder 130 is $F_2$, $F_1 = F_2$. The force $F_1$ of the nitrogen spring is transferred to the fixing cylinder 130 in the direction of the axis of rotation, and the fixing cylinder 130 transfers the force $F_2$ in the direction parallel to the axis via the steel ball.

Under the action of an external force, the rotating part 111 is rotated, and the support frame 112 of the rotating part 111 urges the steel ball to rotate about the axis of rotation A-A along the motion trajectory 131. Since the motion trajectory 131 of the fixing cylinder 130 is inclined (the inclination angle of the motion trajectory 131 is set according to the Formula (2)) and the force-applying mechanism 140 is elastic, the fixing cylinder 130 is urged to move to the right or the left axially and periodically along the guide post 150 when the steel ball is rotated, so that a relative movement is formed between the fixing cylinder 130 and the rotating part 111 while the nitrogen spring is urged to move elastically. The thrust force F applied to the fixing cylinder 130 is a relatively stable force during the elastic movement of the nitrogen spring.

Take the low point M as the origin of the coordinate, the rotation angle α of the centre of gravity of the load as the abscissa, and the compression stroke of the gas spring (i.e., the axial distance between the gas spring and the origin) as the ordinate. As shown in FIG. 8 and FIG. 9, a schematic diagram of a curve relationship between the rotation angle $\alpha$ (in the unit of °) of the centre of gravity of the load and the compression stroke (in the unit of mm) of the gas spring is illustrated. FIG. 9 also shows the forces exerted on the steel ball, including both direction and magnitude.

As shown in FIGs. 8 and 9, when the steel ball rotates by a turn (i.e., 360°) about the motion trajectory 131, the relationship curve is an approximately inverted V-shape. The approximately inverted V-curve is axisymmetric and may form a closed loop when it is connected from one end to the other end. Where the inclining rate of the curve reflects a magnitude of the inclination angle $\beta$ of the motion trajectory 131.

Referring to FIGs. 8 and 9, when $\alpha = 0°$ (or 360°) and 180°, $\beta = 0°$, i.e., the complement angle $\beta$ of the included angle between the tangent of the motion trajectory 131 and the axis of the fixing cylinder 130 is 0°. When $\alpha = 90°$ and 270°, the complement angle $\beta$ of the included angle between the tangent of the motion trajectory 131 and the axis of the fixing cylinder 130 is the largest.

In conjunction with the Formulas (2) and (3), the thrust force F and the support force of the surface of the motion trajectory 131 whose inclination angle is $\beta$ generate a resultant force $F \times \tan\beta$. The resultant force $F \times \tan\beta$ will generate a moment $M_F$, which is calculated according to the formula:

$$M_F = F \times \tan\beta \times r = m \times g \times L \times \sin\alpha \quad (4).$$

In the first and the fourth quadrants, the torque $M_G$ generated by the gravity mg of the load is clockwise and its rotation angle $\alpha$ corresponds to 360° to 180° in FIG. 8 and FIG. 9. Referring to FIG. 9, from 360° to 180°, the torque $M_F$ generated by the resultant force of the thrust force F and the support force is in the direction opposite to $M_G$ and is counter-clockwise. By the calculation according to the Formula (4), it may be seen that $M_F$ is equal to $M_G$ in magnitude, therefore the torque $M_G$ generated by the gravity of the load is counteracted.

Similarly, in the second and third quadrants, the torque $M_G$ generated by the gravity mg of the load is counter-clockwise and its rotation angle $\alpha$ corresponds to 180° to 0° in FIG. 8 and FIG. 9. Referring to FIG. 9, from 180° to 0°, the thrust force F and the support force of the surface of the motion trajectory 131 whose inclination angle is $\beta$ generate a resultant force. The torque $M_F$ generated by the resultant force is in the direction opposite to $M_G$ and is clockwise. By the calculation according to the Formula (4), it may be seen that $M_F$ is equal to $M_G$ in magnitude, therefore the torque $M_G$ generated by the gravity of the load is counteracted.

It may be seen that the thrust force F and the support force of the surface of the motion trajectory 131 whose inclination angle is $\beta$ may generate a resultant force. The torque $M_F$ generated by the resultant force is equal to the torque $M_G$ generated by the gravity of the load in magnitude and is in the direction opposite to the torque $M_G$ generated by the gravity of the load so that the torque $M_G$ generated by the gravity of the load is counteracted and the rotation of the load is balanced, thereby achieving the effect of torque assisting.

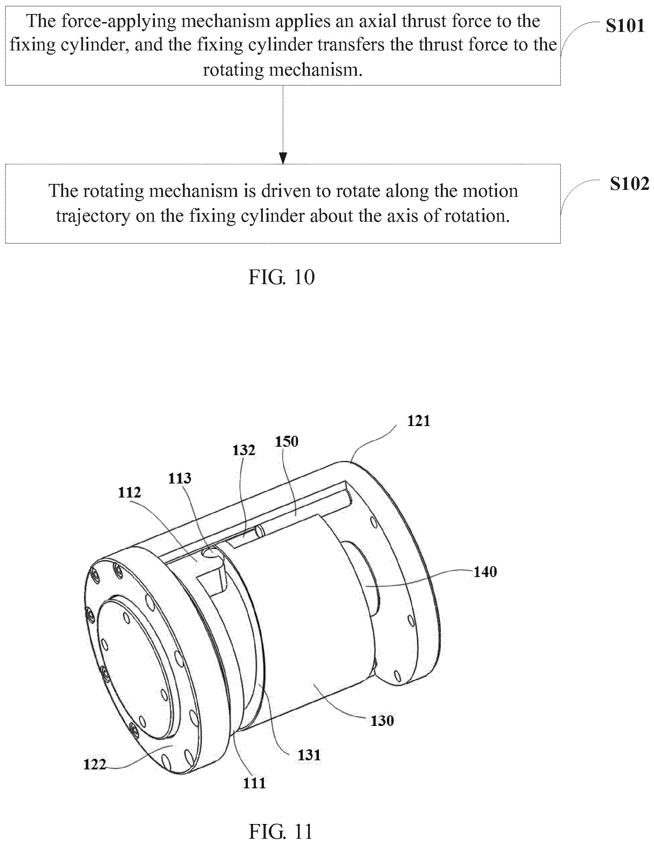

FIG. 10 shows a flow chart of a torque assisting method of the above torque assisting device according to an example of the present disclosure. The torque assisting method includes the following blocks.

S101: The force-applying mechanism 140 applies an axial thrust force F to the fixing cylinder 130, and the fixing cylinder 130 transfers the thrust force to the rotating mechanism 110.

Where the thrust force F is a linear force, and the thrust force F is regular in magnitude and remains relatively stable.

S102: The rotating mechanism 110 is driven to rotate along the motion trajectory 131 on the fixing cylinder 130 about the axis of rotation A-A. In an initial position, the steel ball is in contact with the low point M on the motion trajectory 131, and now the force-applying mechanism is in the maximum extended state.

References are made in the order of FIG. 11, FIG. 12 and FIG. 13, which disclose perspective positional relationships of different components of the torque assisting device when the steel ball is moved from the low point M to the high point N. The rotating mechanism 110 rotates only about the axis of rotation without axially moving. However, since the motion trajectory 131 in contact with the steel ball has a varying inclination angle, the fixing cylinder 130 will be urged to move axially to the right and the force-applying mechanism 140 will be compressed to move axially to the right when the steel ball rotates about the axis of rotation.

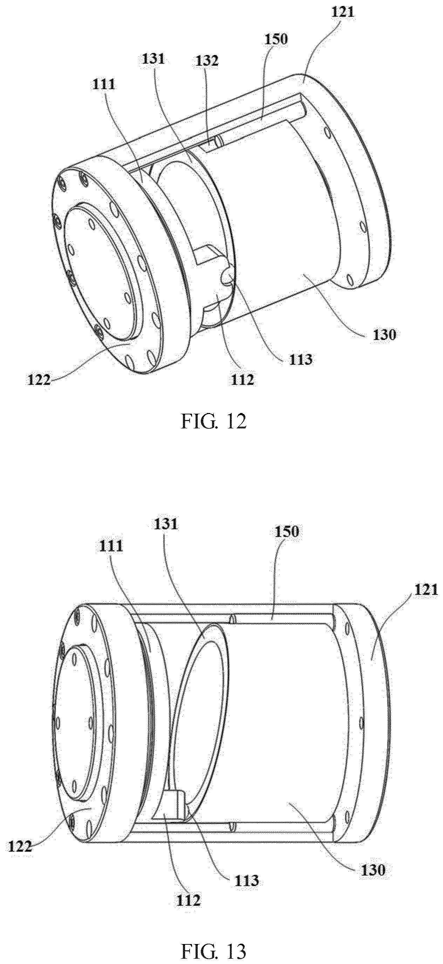

References are also made in the order of FIG. 13, FIG. 14 and FIG. 11, which disclose perspective positional relationships of different components of the torque assisting device when the steel ball is moved from the high point N to the low point M. The rotating mechanism 110 rotates only about the axis of rotation without axially moving. However, since the motion trajectory 131 in contact with the steel ball has a varying inclination angle and the force-applying mechanism 140 applies a thrust force, the force-applying mechanism 140 will urge the fixing cylinder 130 to move axially to the left and the force-applying mechanism 140 will also move axially to the left when the steel ball rotates about the axis of rotation.

When the steel ball is moved from the low point M to the high point N or from the high point N to the low point M, the rotating mechanism 110 is rotated, and the thrust force F is brought into contact with the motion trajectory 131 by the transferring action of the fixing cylinder 130. The thrust force F and the support force of the surface of the motion trajectory 131 whose inclination angle is $\beta$ generate a resultant force $F \times \tan\beta$. The resultant force and r will generate a torque $M_F$ which is equal to the torque $M_G$ generated by the gravity of the load in magnitude and is opposite in the direction so that the torque $M_G$ generated by the gravity of the load is counteracted and the load is rotated in a balanced manner.

In present disclosure, the torque $M_F$ is generated due to the thrust force F of the force-applying mechanism, the inclination angle β of the motion trajectory 131 of the steel ball, and the stress position (radius r) of the steel ball. The torque $M_F$ and the torque $M_G$ generated by the gravity of the load are made approximately equal in value and opposite in direction by designing the value of β to counteract the torque $M_G$ generated by the gravity of the load when the load is rotated about the axis (i.e., the axis of rotation A-A).

In summary, a torque associated with the rotation angle is output passively by setting the rotating mechanism to rotate along a preset motion trajectory on the fixing cylinder about the axis of rotation and the fixing cylinder capable of transferring a force, thereby counteracting the torque generated by the gravity of the load when the rotating mechanism rotates about the axis, maintaining the balance of rotation of the load, lowering the driving requirements for the rotating mechanism, and improving the accuracy with which the rotating mechanism is controlled to rotate about the axis and the safety of the device. The torque assisting device is particularly suitable for the application scenario where a rigid object (i.e., a load) generates a torque due to its gravity when rotating at a medium-low speed about an axis.

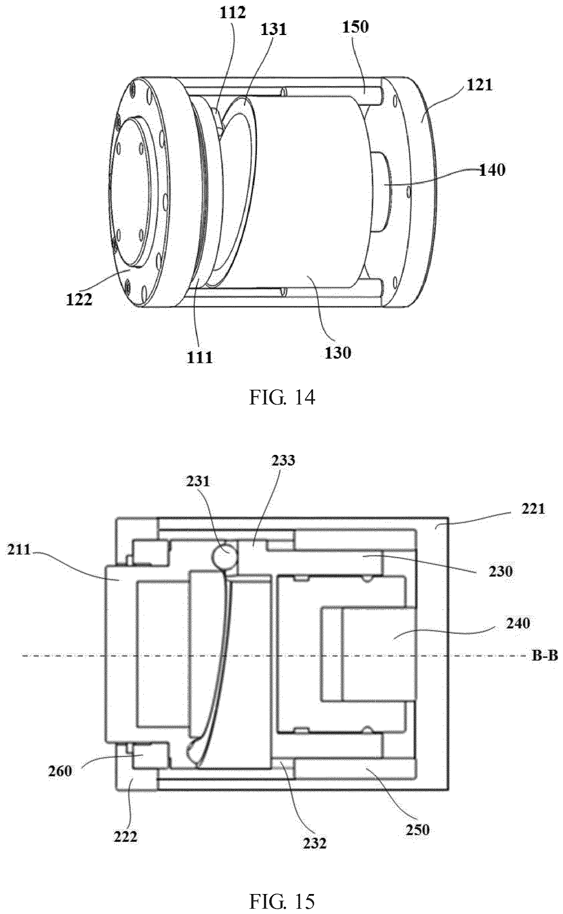

Because $M_F$ is generated depending on the relative movement, the motion trajectory of the relative movement and the position of the steel ball may be interchangeable. For example, the motion trajectory is provided on the rotating part, and the steel ball is provided on the fixing cylinder. FIG. 15 shows a cross-sectional diagram of a torque assisting device according to another example.

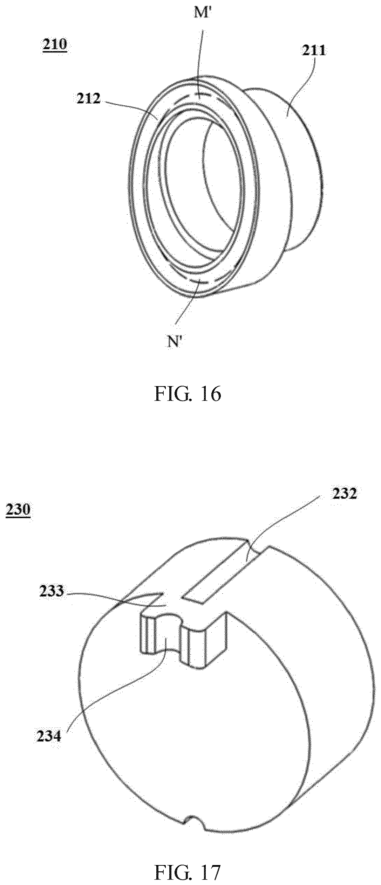

In conjunction with FIGs. 15 to 17, another torque assisting device according to the present disclosure includes a rotating mechanism 210, a fixing cylinder 230, and a force-applying mechanism 240, where the rotating mechanism 210 is provided with a load (not shown in the drawings). The rotating mechanism 210 has one end provided with a motion trajectory 212 having an inclination angle which is set according to the torque generated by the gravity of the load. The fixing cylinder 230 is abutted on the motion trajectory 212, and the fixing cylinder 230 is connected with the force-applying mechanism 240. Alternatively, one end of the fixing cylinder 230 is abutted on the motion trajectory 212 and the other end thereof is connected with the force-applying mechanism 240. The force-applying mechanism 240 may be elastic under an external force, i.e., the force-applying mechanism 240 may be moved axially.

In an example, the fixing cylinder 230 is provided with a support frame 233. The support frame 233 is provided with a rigid member 231. The rigid member 231 is in a point contact or a line contact with the motion trajectory 212 to achieve a transfer of a force, thereby urging the fixing cylinder 230 to move axially and compressing the force-applying mechanism.

The rigid member 213 may be a steel ball, a roller or a non-roller body. The present example will be further described with a steel ball as the rigid member 213.

The support frame 233 may protrude from the rotating part 211 to prevent the rotating part 211 from colliding with the fixing cylinder 230 during rotation.

Alternatively, the fixing cylinder 230 and the support frame 233 may be integrally molded, so that the fixing cylinder 230 is simpler in structure.

To better fix the steel ball and prevent the steel ball from falling, the support frame 233 is provided with a clamping groove 234 for clamping the steel ball.

In an example, the inclination angle β of the motion trajectory 212 on the rotating mechanism 210 is:

$$\beta = \arctan\left(\frac{m \times g \times L \times \sin\alpha}{F \times r}\right)$$

where m is the mass of a load;

g is gravitational acceleration;
L is a vertical distance from the centre of gravity of the load to an axis of rotation B-B;
α is the rotation angle of the centre of gravity of the load;
r is a vertical distance from the motion trajectory 212 to the axis of rotation B-B; and
F is an axial thrust force applied to the fixing cylinder 230 by the force-applying mechanism 240. The fixing cylinder 230 may transfer the thrust force to the rotating mechanism 210, i.e., the thrust force acts on the contact position of the rotating mechanism 210 and the fixing cylinder 230. Herein the thrust force F varies regularly, for example, the thrust force F is a linearly varying force. The vertical distance from the action point of the thrust force to the axis of rotation B-B is equal to the vertical distance from the motion trajectory 212 to the axis of rotation B-B.

In an example, the fixing cylinder 230 is a cylinder, and the centre axis thereof is the axis of rotation B-B. The steel ball is provided on the fixing cylinder 230 and is abutted on the motion trajectory 212 located at one end of the rotating mechanism 210.

In an initial position, the steel ball is in contact with the low point M' on the motion trajectory 212, and now the force-applying mechanism may be in the maximum extended state.

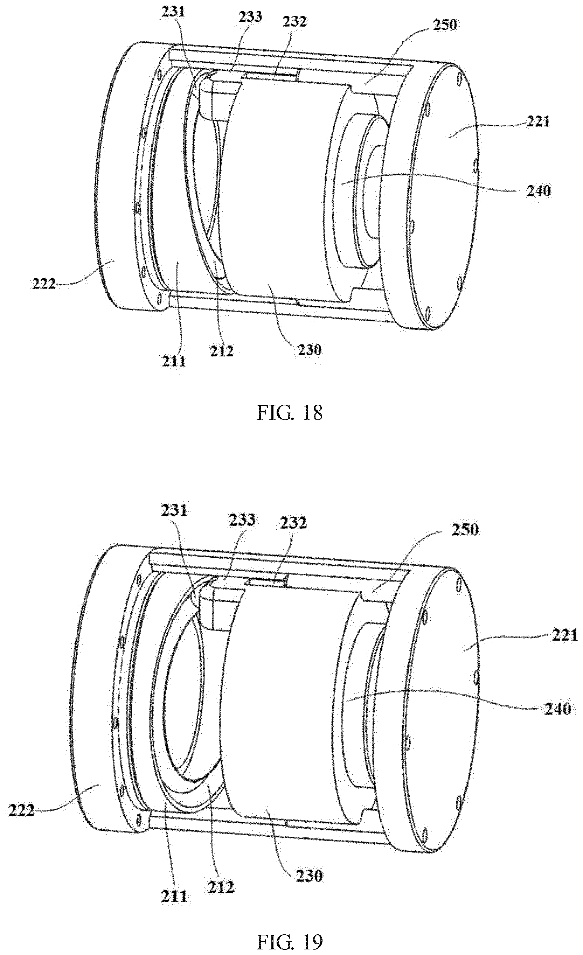

References are made in the order of FIG. 18, FIG. 19 and FIG. 20, which disclose the perspective positional relationships of different components of the torque assisting device when the steel ball is moved from the low point M' to the high point N'. The rotating mechanism 210 rotates only about the axis of rotation without axially moving. However, since the motion trajectory 212 in contact with the steel ball has a varying inclination angle, the fixing cylinder 230 will be urged to move axially to the right and the force-applying mechanism 240 will be compressed to move axially to the right when the motion trajectory 212 rotates about the axis of rotation B-B.

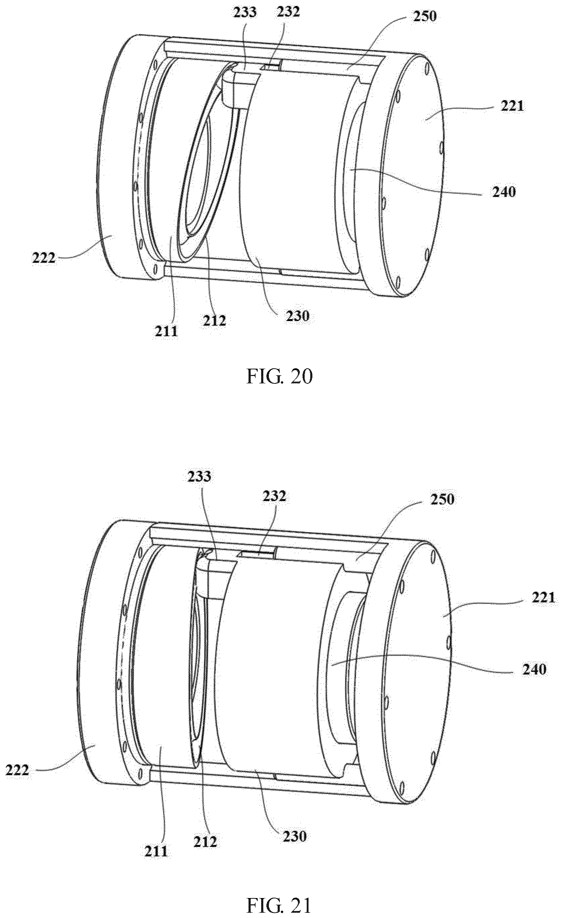

References are also made in the order of FIG. 20, FIG. 21 and FIG. 18, which disclose the perspective positional relationships of different components of the torque assisting device when the steel ball is moved from the high point N' to the low point M'. The rotating mechanism 210 rotates only about the axis of rotation without axially moving. However, since the motion trajectory 212 in contact with the steel ball has a varying inclination angle and the force-applying mechanism 240 applies a thrust force, the force-applying mechanism 240 will urge the fixing cylinder 230 to move axially to the left and the force-applying mechanism 240 will also move axially to the left when the motion trajectory 212 rotates about the axis of rotation.

When the steel ball is moved from the low point M' to the high point N' or from the high point N' to the low point M', the rotating mechanism 210 is rotated, and the motion trajectory 212 on the rotating mechanism 210 is rotated about the axis of rotation B-B to urge the fixing cylinder 230 to move axially, so that a relative movement is formed between the fixing cylinder 230 and the rotating part 211 while the force-applying mechanism 240 is urged to move elastically.

When the rotating mechanism 210 is rotated, the thrust force F is brought into contact with the motion trajectory 212 by the transferring action of the fixing cylinder 230. The thrust force F and the support force of the surface of the motion trajectory 212 whose inclination angle is $\beta$ generate a resultant force $F \times \tan\beta$. The resultant force and r will generate a torque $M_F$. The torque $M_F$ and the torque $M_G$ generated by the gravity of the load are equal in magnitude and opposite in direction so that the torque $M_G$ generated by the gravity of the load is counteracted and the load is rotated in a balanced manner.

It may be seen that the motion trajectory 212 actively rotates in this example while the steel ball actively rotates in the former example.

The torque assisting device of the present example further includes a cylinder body 221, a cylinder head 222, a guide groove 232, a guide post 250, a bearing 260, etc. These components are provided in the same manner as the former example, which will not be described again.

It is to be noted that the difference between the former example and the latter example only lies in the fixing position of a steel ball, the setting position of a motion trajectory and the change of an actively-rotating part (the steel ball or the motion trajectory). The latter example and the former example are the same in other aspects. The former example may be referred to for details, which will not be described again.

In summary, the torque associated with the rotation angle is output passively by setting the motion trajectory having an inclination angle of the rotating mechanism to rotate about the axis of rotation and the fixing cylinder capable of transferring a force, thereby counteracting the torque generated by the gravity of the load when the rotating mechanism rotates about the axis of rotation, maintaining the balance of rotation of the load, lowering the driving requirements for the rotating mechanism, and improving the accuracy with which the rotating mechanism is controlled to rotate about the axis and the safety of the device. The torque assisting device is particularly suitable for the application scenario where a rigid object (i.e., a load) generates a torque due to its gravity when rotating at a medium-low speed about an axis.

The foregoing is intended only as a preferred example of the present disclosure and is not intended to be limiting the present disclosure. Any modifications, equivalent substitutions, improvements, etc., which are made within the principle of the present disclosure, shall fall in the scope of protection of the present disclosure.

For simplicity and illustrative purposes, the present disclosure is described by referring mainly to an example thereof. In the following description, numerous specific details are set forth to provide a thorough understanding of the present disclosure. It will be clear however, that the present disclosure may be practiced without limitation to these specific details. In other instances, some methods and structures have not been described in detail so as not to unnecessarily obscure the present disclosure. As used herein, the terms "a" and "an" are intended to denote at least one particular element, the term "includes" means includes but not limited to, the term "including" means including but not limited to, and the term "based on" means based at least in part on.

Throughout the present disclosure, the word "include", or variations such as "includes" or "including", will be understood to imply the inclusion of a stated element, integer or block, or group of elements, integers or blocks, but not the exclusion of any other element, integer or block, or group of elements, integers or blocks.

It will be appreciated by persons skilled in the art that numerous variations and/or modifications may be made to the above-described examples, without departing from the broad general scope of the present disclosure. The present examples are, therefore, to be considered in all respects as illustrative and not restrictive.

**Claims**

1. A torque assisting device, **characterized in that** the device comprises:

   a rotating mechanism (110) on which a load is provided;

a fixing cylinder (130) of which a circumference of one end surface is provided with a continuous motion trajectory (131) having an inclination angle; and

an axially elastic force-applying mechanism (140) connected with the fixing cylinder (130), wherein one end of the rotating mechanism (110) is abutted on the continuous motion trajectory (131) and the rotating mechanism (110) rotates about an axis of rotation (A-A) along the continuous motion trajectory (131); and

the inclination angle of the continuous motion trajectory (131) is set according to a torque generated by the gravity of the load based on the following equation:

$$\beta = \arctan\left(\frac{m \times g \times L \times \sin\alpha}{F \times r}\right)$$

wherein m is a mass of the load,
g is the gravitational acceleration,
L is a vertical distance from the centre of gravity of the load to the axis of rotation (A-A),
$\alpha$ is a rotation angle of the centre of gravity of the load,
r is a vertical distance from the continuous motion trajectory (131) to the axis of rotation (A-A), and
F is an axial thrust force applied to the abutting point between the rotating mechanism (110) and the continuous motion trajectory (131) by the force-applying mechanism (140), and a vertical distance from an action point of the thrust force to the axis of rotation (A-A) is equal to a vertical distance from the continuous motion trajectory (131) to the axis of rotation (A-A).

2. The torque assisting device according to claim 1, wherein the continuous motion trajectory (131) is provided with a trajectory groove engaged with the rotating mechanism (110).

3. The torque assisting device according to claim 1, wherein the device further comprises a guide post (150),
wherein the fixing cylinder (130) is provided with a guide groove (132) slidably engaged with the guide post (150) in a way that the fixing cylinder (130) is moved axially.

4. The torque assisting device according to claim 3, wherein there are at least two guide posts (150) which are evenly distributed along a circumference of the fixing cylinder (130).

5. The torque assisting device according to claim 3, wherein the device further comprises a cylinder body (121), a cylinder head (122) and a bearing (160);
wherein the guide post (150) and the force-applying mechanism (140) are fixedly connected with an inner wall of the cylinder body (121), and
the rotating mechanism (110) is fixedly connected with the bearing (160).

6. The torque assisting device according to claim 1, wherein the force-applying mechanism (140) is a gas spring.

7. The torque assisting device according to claim 1, wherein the rotating mechanism (110) comprises:

a rotating part (111);
a support frame (112) provided on the rotating part (111); and
a rigid member (113) fixed to the support frame (112),
wherein the rigid member (113) is in a point contact or a line contact with the continuous motion trajectory (131).

8. The torque assisting device according to claim 7, wherein the rotation angle of the centre of gravity of the load is equal to the rotation angle of the rigid member.

9. A torque assisting method suitable for the torque assisting device according to claim 1, **characterized in that** the method comprises:

applying, by the force-applying mechanism (140), an axial thrust force to the fixing cylinder (130); and
transferring, by the fixing cylinder (130), the thrust force to the rotating mechanism (110) in a way that the rotating mechanism (110) is driven to rotate about the axis of rotation (A-A) along the continuous motion trajectory (131) on the fixing cylinder (130).

10. A mammary machine, **characterized in that** the machine comprises the torque assisting device according to claim 1, wherein the load is a rotating part of the mammary machine.

11. A torque assisting device, **characterized in that** the device comprises:

a rotating mechanism (210) on which a load and a continuous motion trajectory (212) having an inclination angle are provided;
a fixing cylinder (230) of which one end is abutted on the continuous motion trajectory (212); and

an axially elastic force-applying mechanism (240) connected with the fixing cylinder (230), wherein the rotating mechanism (210) rotates about an axis of rotation (B-B); and

the inclination angle of the continuous motion trajectory (212) is set according to a torque generated by a gravity of the load based on the following equation:

$$\beta = \arctan\left(\frac{m \times g \times L \times \sin\alpha}{F \times r}\right)$$

wherein m is a mass of the load,

g is the gravitational acceleration,

L is a vertical distance from the centre of gravity of the load to the axis of rotation (A-A),

α is a rotation angle of the centre of gravity of the load,

r is a vertical distance from the continuous motion trajectory (212) to the axis of rotation (A-A), and

F is an axial thrust force applied to the abutting point between the rotating mechanism (210) and the continuous motion trajectory (212) by the force-applying mechanism (240), and a vertical distance from an action point of the thrust force to the axis of rotation (A-A) is equal to a vertical distance from the continuous motion trajectory (212) to the axis of rotation (A-A).

12. The torque assisting device according to claim 11, wherein the device further comprises a guide post (250),
wherein the fixing cylinder (230) is provided with a guide groove (232) slidably engaged with the guide post (250) in a way that the fixing cylinder (230) is moved axially.

13. A torque assisting method suitable for the torque assisting device according to claim 11, **characterized in that** the method comprises:

applying, by the force-applying mechanism (240), an axial thrust force to the fixing cylinder (230); and
transferring, by the fixing cylinder (230), the thrust force to the rotating mechanism (210) in a way that the rotating mechanism (210) is driven to rotate about the axis of rotation (B-B) and a rigid member (231) on the fixing cylinder (230) is caused to move along the continuous motion trajectory (212).

14. A mammary machine, **characterized in that** the machine comprises the torque assisting device according to claim 11, wherein the load is a rotating part of the mammary machine.

## Patentansprüche

1. Drehmomentassistenzvorrichtung, **dadurch gekennzeichnet, dass** die Vorrichtung aufweist:

einen Drehmechanismus (110), auf welchem eine Last vorgesehen wird;
einen Fixierzylinder (130), von welchem ein Umfang einer Stirnfläche mit einer kontinuierlichen Bewegungsbahn (131) versehen ist, die einen Neigungswinkel hat; und
einen axial elastischen Kraftausübungsmechanismus (140), der mit dem Fixierzylinder (130) verbunden ist,
wobei ein Ende des Drehmechanismus (110) an der kontinuierlichen Bewegungsbahn (131) anliegt und der Drehmechanismus (110) sich um eine Drehachse (A-A) entlang der kontinuierlichen Bewegungsbahn (131) dreht; und
der Neigungswinkel der kontinuierlichen Bewegungsbahn (131) entsprechend einem Drehmoment, das durch die Schwerkraft der Last hervorgerufen wird, auf der Grundlage der folgenden Gleichung festgelegt ist

$$\beta = \arctan\left(\frac{m \times g \times L \times \sin\alpha}{F \times r}\right)$$

wobei

m die Masse der Last ist,
g die Gravitationsbeschleunigung ist,
L ein vertikaler Abstand vom Schwerpunkt der Last zu der Drehachse (A-A) ist,
α ein Drehwinkel des Schwerpunkts der Last ist,
r ein vertikaler Abstand von der kontinuierlichen Bewegungsbahn (131) zu der Drehachse (A-A) ist, und
F eine axiale Schubkraft ist, die auf den Anlagepunkt zwischen dem Drehmechanismus (110) und der kontinuierlichen Bewegungsbahn (131) durch den Kraftausübungsmechanismus (140) ausgeübt wird, und
ein vertikaler Abstand von einem Wirkungspunkt der Schubkraft zu der Drehachse (A-A) gleich einem vertikalen Abstand von der kontinuierlichen Bewegungsbahn (131) zu der Drehachse (A-A) ist.

2. Drehmomentassistenzvorrichtung nach Anspruch 1, wobei die kontinuierliche Bewegungsbahn (131) mit einer Bahnrille versehen ist, die mit dem Drehmechanismus (110) in Eingriff ist.

3. Drehmomentassistenzvorrichtung nach Anspruch 1, wobei die Vorrichtung ferner einen Führungsstab (150) aufweist,
wobei der Fixierzylinder (130) mit einer Führungsnut (132) versehen ist, die in Gleiteingriff mit dem Führungsstab (150) derart ist, dass der Fixierzylinder (130) axial bewegt wird.

4. Drehmomentassistenzvorrichtung nach Anspruch 3, wobei es mindestens zwei Führungsstäbe (150) gibt, die entlang eines Umfangs des Fixierzylinders (130) gleichmäßig verteilt sind.

5. Drehmomentassistenzvorrichtung nach Anspruch 3, wobei die Vorrichtung ferner einen Zylinderkörper (121), einen Zylinderkopf (122) und ein Lager (160) aufweist;
wobei der Führungsstab (150) und der Kraftausübungsmechanismus (140) fest mit einer Innenwand des Zylinderkörpers (121) verbunden sind, und der Drehmechanismus (110) fest mit dem Lager (160) verbunden ist.

6. Drehmomentassistenzvorrichtung nach Anspruch 1, wobei der Kraftausübungsmechanismus (140) eine Gasfeder ist.

7. Drehmomentassistenzvorrichtung nach Anspruch 1, wobei der Drehmechanismus (110) aufweist:

ein drehendes Teil (111);
einen Halterahmen (112), der an dem drehenden Teil (111) vorgesehen ist; und
ein starres Element (113), das an dem Halterahmen (112) befestigt ist,
wobei das starre Element (113) in Punktkontakt oder Linienkontakt mit der kontinuierlichen Bewegungsbahn (131) steht.

8. Drehmomentassistenzvorrichtung nach Anspruch 7, wobei der Drehwinkel des Schwerpunkts der Last gleich dem Drehwinkel des starren Elements ist.

9. Drehmomentassistenzverfahren, das für die Drehmomentassistenzvorrichtung nach Anspruch 1 geeignet ist, **dadurch gekennzeichnet, dass** das Verfahren umfasst:

Ausüben, durch den Kraftausübungsmechanismus (140), einer axialen Schubkraft auf den Fixierzylinder (130); und
Übertragen, durch den Fixierzylinder (130), der Schubkraft auf den Drehmechanismus (110) derart, dass der Drehmechanismus (110) so angetrieben wird, dass er sich um die Drehachse (A-A) entlang der kontinuierlichen Bewegungsbahn (131) auf dem Fixierzylinder (130) dreht.

10. Brustmaschine, **dadurch gekennzeichnet, dass** die Maschine die Drehmomentassistenzvorrichtung nach Anspruch 1 aufweist, wobei die Last ein drehendes Teil der Brustmaschine ist.

11. Drehmomentassistenzvorrichtung, **dadurch gekennzeichnet, dass** die Vorrichtung aufweist:

einen Drehmechanismus (210), auf welchem eine Last und eine kontinuierliche Bewegungsbahn (212), die einen Neigungswinkel hat, vorgesehen sind;
einen Fixierzylinder (230), dessen eines Ende an der kontinuierlichen Bewegungsbahn (212) anliegt; und
einen axial elastischen Kraftausübungsmechanismus (240), der mit dem Fixierzylinder (230) verbunden ist,
wobei sich der Drehmechanismus (210) um eine Drehachse (B-B) dreht; und
der Neigungswinkel der kontinuierlichen Bewegungsbahn (212) entsprechend einem Drehmoment, das durch eine Schwerkraft der Last hervorgerufen wird, auf der Grundlage der folgenden Gleichung festgelegt ist:

$$\beta = \arctan\left(\frac{m \times g \times L \times \sin\alpha}{F \times r}\right)$$

wobei m die Masse der Last ist,
g die Gravitationsbeschleunigung ist,
L ein vertikaler Abstand von dem Schwerpunkt der Last zu der Drehachse (B-B) ist,
$\alpha$ der Drehwinkel des Schwerpunkts der Last ist,
r ein vertikaler Abstand von der kontinuierlichen Bewegungsbahn (212) zu der Drehachse (B-B) ist, und
F eine axiale Schubkraft ist, die auf den Anlagepunkt zwischen dem Drehmechanismus (210) und der kontinuierlichen Bewegungsbahn (212) durch den Kraftausübungsmechanismus (240) ausgeübt wird, und wobei ein vertikaler Abstand von einem Wirkungspunkt der Schubkraft auf die Drehachse (B-B) gleich einem vertikalen Abstand von der kontinuierlichen Bewegungsbahn (212) zu der Drehachse (B-B) ist.

12. Drehmomentassistenzvorrichtung nach Anspruch 11, wobei die Vorrichtung ferner einen Führungsstab (250) aufweist,
wobei der Fixierzylinder (230) mit einer Führungsnut (232) versehen ist, die mit dem Führungsstab (250) derart gleitend in Eingriff ist, dass der Fixierzylinder (230) axial bewegt wird.

13. Drehmomentassistenzverfahren, das für die Drehmomentassistenzvorrichtung nach Anspruch 11 geeignet ist, **dadurch gekennzeichnet, dass** das Verfahren umfasst:

Ausüben, durch den Kraftausübungsmechanismus (240), einer axialen Schubkraft auf den Fixierzylinder (230); und
Übertragen, durch den Fixierzylinder (230), der Schubkraft auf den Drehmechanismus (210) derart, dass der Drehmechanismus (210) so angetrieben wird, dass er sich um die Drehachse (B-B) dreht, und ein starres Element (231) auf dem Fixierzylinder (230) veranlasst wird, sich entlang der kontinuierlichen Bewegungsbahn (212) zu bewegen.

14. Brustmaschine, **dadurch gekennzeichnet, dass** die Maschine die Drehmomentassistenzvorrichtung nach Anspruch 11 aufweist, wobei die Last ein drehendes Teil der Brustmaschine ist.

## Revendications

1. Dispositif d'assistance au couple, **caractérisé en ce que** le dispositif comprend :

un mécanisme de rotation (110) sur lequel une charge est prévue ;
un cylindre de fixation (130) dont une circonférence d'une surface d'extrémité est munie d'une trajectoire de mouvement continu (131) ayant un angle d'inclinaison ; et
un mécanisme d'application de force axialement élastique (140) relié au cylindre de fixation (130), où une extrémité du mécanisme de rotation (110) vient en butée sur la trajectoire de mouvement continu (131) et le mécanisme de rotation (110) tourne autour d'un axe de rotation (A-A) le long de la trajectoire de mouvement continu (131) ; et
l'angle d'inclinaison de la trajectoire de mouvement continu (131) est défini selon un couple généré par la gravité de la charge basée sur l'équation suivante :

$$\beta = \arctan\left(\frac{m \times g \times L \times \sin\alpha}{F \times r}\right)$$

où m est une masse de la charge,
g est l'accélération gravitationnelle,
L est une distance verticale depuis le centre de gravité de la charge vers l'axe de rotation (A-A),
α est un angle de rotation du centre de gravité de la charge,
r est une distance verticale depuis la trajectoire de mouvement continu (131) vers l'axe de rotation (A-A), et
F est une force de poussée axiale appliquée au point de butée entre le mécanisme de rotation (110) et la trajectoire de mouvement continu (131) par le mécanisme d'application de force (140), et une distance verticale depuis un point d'action de la force de poussée vers l'axe de rotation (A-A) est égale à une distance verticale depuis la trajectoire de mouvement continu (131) vers l'axe de rotation (A-A).

2. Dispositif d'assistance au couple selon la revendication 1, la trajectoire de mouvement continu (131) étant munie d'une rainure de trajectoire engagée avec le mécanisme de rotation (110).

3. Dispositif d'assistance au couple selon la revendication 1, le dispositif comprenant en outre un montant de guidage (150),
où le cylindre de fixation (130) est muni d'une rainure de guidage (132) engagée de manière coulissante avec le montant de guidage (150) d'une manière telle que le cylindre de fixation (130) est axialement déplacé.

4. Dispositif d'assistance au couple selon la revendication 3, dans lequel au moins deux montants de guidage (150) sont présents qui sont répartis de manière uniforme le long d'une circonférence du cylindre de fixation (130).

5. Dispositif d'assistance au couple selon la revendication 3, le dispositif comprenant en outre un corps de cylindre (121), une tête de cylindre (122) et un roulement (160) ;
le montant de guidage (150) et le mécanisme d'application de force (140) étant reliés de manière fixe à une paroi interne du corps de cylindre (121), et le mécanisme de rotation (110) étant relié de manière fixe au roulement (160).

6. Dispositif d'assistance au couple selon la revendication 1, le mécanisme d'application de force (140) étant un ressort à gaz.

7. Dispositif d'assistance au couple selon la revendication 1, le mécanisme de rotation (110) comprenant :

une pièce rotative (111) ;
un châssis de support (112) disposé sur la pièce rotative (111) ; et

un élément rigide (113) fixé au châssis de support (112),

où l'élément rigide (113) se trouve en un contact ponctuel ou un contact linéaire avec la trajectoire de mouvement continu (131).

8. Dispositif d'assistance au couple selon la revendication 7, l'angle de rotation du centre de gravité de la charge étant égal à l'angle de rotation de l'élément rigide.

9. Procédé d'assistance au couple convenant au dispositif d'assistance au couple selon la revendication 1, **caractérisé en ce que** le procédé comprend :

l'application, par le mécanisme d'application de force (140), d'une force de poussée axiale au cylindre de fixation (130) ; et

le transfert, par le cylindre de fixation (130), de la force de poussée au mécanisme de rotation (110) d'une manière telle que le mécanisme de rotation (110) est entraîné à tourner autour de l'axe de rotation (A-A) le long de la trajectoire de mouvement continu (131) sur le cylindre de fixation (130).

10. Machine mammaire, **caractérisée en ce que** la machine comprend le dispositif d'assistance au couple selon la revendication 1, la charge étant une pièce rotative de la machine mammaire.

11. Dispositif d'assistance au couple, **caractérisé en ce que** le dispositif comprend :

un mécanisme de rotation (210) sur lequel une charge et une trajectoire de mouvement continu (212) ayant un angle d'inclinaison sont prévues ;
un cylindre de fixation (230) dont une extrémité vient en butée sur la trajectoire de mouvement continu (212) ; et
un mécanisme d'application de force axialement élastique (240) relié au cylindre de fixation (230), où le mécanisme de rotation (210) tourne autour d'un axe de rotation (B-B) ; et
l'angle d'inclinaison de la trajectoire de mouvement continu (212) est défini selon un couple généré par une gravité de la charge basée sur l'équation suivante :

$$\beta = \arctan(\frac{m \times g \times L \times \sin\alpha}{F \times r})$$

où m est une masse de la charge,
g est l'accélération gravitationnelle,
L est une distance verticale depuis le centre de gravité de la charge vers l'axe de rotation (A-A),
α est un angle de rotation du centre de gravité de la charge,
r est une distance verticale depuis la trajectoire de mouvement continu (212) vers l'axe de rotation (A-A), et
F est une force de poussée axiale appliquée au point de butée entre le mécanisme de rotation (210) et la trajectoire de mouvement continu (212) par le mécanisme d'application de force (240), et une distance verticale depuis un point d'action de la force de poussée vers l'axe de rotation (A-A) est égale à une distance verticale depuis la trajectoire de mouvement continu (212) vers l'axe de rotation (A-A).

12. Dispositif d'assistance au couple selon la revendication 11, le dispositif comprenant en outre un montant de guidage (250),
où le cylindre de fixation (230) est muni d'une rainure de guidage (232) engagée de manière coulissante avec le montant de guidage (250) d'une manière telle que le cylindre de fixation (230) est axialement déplacé.

13. Procédé d'assistance au couple convenant au dispositif d'assistance au couple selon la revendication 11, **caractérisé en ce que** le procédé comprend :

l'application, par le mécanisme d'application de force (240), d'une force de poussée axiale au cylindre de fixation (230) ; et

le transfert, par le cylindre de fixation (230), de la force de poussée au mécanisme de rotation (210) d'une manière telle que le mécanisme de rotation (210) est entraîné à tourner autour de l'axe de rotation (B-B) et un élément rigide (231) sur le cylindre de fixation (230) est amené à se déplacer le long de la trajectoire de mouvement continu (212).

14. Machine mammaire, **caractérisée en ce que** la machine comprend le dispositif d'assistance au couple selon la revendication 11, la charge étant une pièce rotative de la machine mammaire.

FIG. 1

FIG. 2

110

111

112

114

FIG. 3

130

132

M

131

N

FIG. 4

FIG. 5

FIG. 6

FIG. 7

FIG. 8

FIG. 9

| The force-applying mechanism applies an axial thrust force to the fixing cylinder, and the fixing cylinder transfers the thrust force to the rotating mechanism. | S101 |

| The rotating mechanism is driven to rotate along the motion trajectory on the fixing cylinder about the axis of rotation. | S102 |

FIG. 10

FIG. 11

FIG. 12

FIG. 13

FIG. 14

FIG. 15

FIG. 16

FIG. 17

FIG. 18

FIG. 19

FIG. 20

FIG. 21

**EP 3 311 747 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011062837 A1
- US 2015145397 A1
- US 5109571 A
- US 5388141 A